# EUROPEAN PATENT SPECIFICATION

(11) **EP 4 107 145 B1**
(45) Date of publication and mention of the grant of the patent: **02.08.2023**
(21) Application number: 21709333.5
(22) Date of filing: 03.02.2021
(51) Int. Cl.: C07D 403/06, A61K 41/00, A61P 31/04, A61P 35/00

(54) **PYRAZOLE-INDOLE CONJUGATES FOR PHOTODYNAMIC TREATMENT OF CANCER AND BACTERIAL INFECTIONS**
PYRAZOL-INDOL-KONJUGATE ZUR FOTODYNAMISCHEN BEHANDLUNG VON KREBS UND BAKTERIELLEN INFEKTIONEN
CONJUGUÉS PYRAZOLE-INDOLE POUR LE TRAITEMENT PHOTODYNAMIQUE DU CANCER ET D'INFECTIONS BACTÉRIENNES

(30) Priority: 18.02.2020 CZ 20200078
(43) Date of publication of application: 28.12.2022
(73) Proprietor: Univerzita Palackého v Olomouci, 779 00 Olomouc (CZ); Kauno Technologijos Universitetas, 44249 Kaunas (LT)
(72) Inventor: VARVUOLYTE, Gabriele, 53180 Margininku kaimas, Kauno raj. (LT); MALINA, Lukas, 78501 Stemberk (CZ); BIELIAUSKAS, Aurimas, 46391 Kaunas (LT); HOSIKOVA, Barbora, 79604 Prostejov (CZ); SIMERSKA, Helena, 77900 Olomouc (CZ); KOLAROVA, Hana, 78347 Hnevotin (CZ); KLEIZIENE, Neringa, 48147 Kaunas (LT); STRNAD, Miroslav, 77900 Olomouc (CZ); ARBACIAUSKIENE, Egle, 44282 Kaunas (LT); KRYSTOF, Vladimir, 77900 Olomouc (CZ); SACKUS, Algirdas, 44326 Kaunas (LT); ZUKAUSKAITE, Asta, 77900 Olomouc (CZ)
(74) Representative: Harber IP s.r.o.
(86) International application number: PCT/CZ2021/050013
(87) International publication number: WO 2021/164795

(56) References cited:
- US-A1- 2016 213 780
- DOS SANTOS A FERREIRA ET AL: "Photodynamic therapy in cancer treatment - an update review", JOURNAL OF CANCER METASTASIS AND TREATMENT, vol. 2019, 29 March 2019 (2019-03-29), XP055799452, ISSN: 2394-4722, DOI: 10.20517/2394-4722.2018.83
- ZIMINOV ANDREY V ET AL: "Synthesis, characterization, and investigation of photochemical properties of tetra-substituted zinc phthalocyanines bearing 4-(3,5-dimethyl-1H-pyrazol-1-yl)phenyl moiety with different linker heteroatoms", INORGANICA CHIMICA ACTA, ELSEVIER BV, NL, vol. 501, 28 November 2019 (2019-11-28), XP085971354, ISSN: 0020-1693, DOI: 10.1016/J.ICA.2019.119306 [retrieved on 2019-11-28]
- KREIZA GEDIMINAS ET AL: "Fluorescence sensing based on phenylenediacetonitrile doped into polymer host", JOURNAL OF LUMINESCENCE, ELSEVIER BV NORTH-HOLLAND, NL, vol. 170, 6 November 2015 (2015-11-06), pages 293-298, XP029328579, ISSN: 0022-2313, DOI: 10.1016/J.JLUMIN.2015.10.040
- VARVUOLYTE GABRIELE ET AL: "Synthesis and photodynamic properties of pyrazole-indole hybrids in the human skin melanoma cell line G361", DYES AND PIGMENTS, vol. 183, 12 July 2020 (2020-07-12), page 108666, XP055799411, GB ISSN: 0143-7208, DOI: 10.1016/j.dyepig.2020.108666

## Description

### Field of the Invention

The present invention relates to new conjugated pyrazole-indole derivatives, which possess low dark toxicity while exhibiting photodynamic properties against cancer cell lines and bacteria under light conditions. The invention also describes a method of production of said new compounds and intermediates for preparation thereof, and use of the new compounds as medicaments.

### Background Art

Photodynamic therapy (PDT) is a non-surgical, minimally invasive treatment which uses light-sensitive medication (photosensitizer, PS), light source and oxygen to produce cytotoxic reactive oxygen species (ROS), which in turn cause abnormal cell destruction (Tampa et al., Oncol. Lett. 17 (2019) 4085). Nowadays, several skin conditions, such as psoriasis, acne, bacterial infections, as well as various types of cancer such as basal cell carcinoma, squamous cell carcinoma, melanoma, lung, head, mouth, nasopharyngeal, laryngeal, bladder, vaginal or cervical cancers can be treated using PDT (Svanberg and Bendsoe, Lasers Med. Appl. (2013) 760; Usuda et al., J. Thorac. Oncol. 1 (2006) 489).

Clinically used photosensitizers are typically macromolecular porphyrinoid compounds, such as porphyrins, chlorins, bacteriochlorins, phthalocyanines, for instance porfimer sodium (HPD), mono-Z-aspartyl chlorin e6 (NPe6), palladium bactereopheophorbide (WST-11), temoporfin, verteporfin, redaporfin (Dabrowski et al., Photobiol. Sci. 14 (2015) 1765-1780; Dos Santos et al., J. Cancer Metastasis Treat. 5 (2019) 25). or precursors of endogenous protoporphyrin IX (PpIX), i.e. δ-aminolevulinic acid (ALA), methyl aminolevulinate (MAL) and hexaminolevulinate (HAL).

On the other hand, variety of other compounds with prolonged conjugated systems have been revealed to possess photodynamic properties, for instance, boron dipyrromethenes, also known as BODIPYs (Zhang et al., Acta Pharm. Sin. B. 8 (2018) 137), their analogues aza-BODIPYs (Awuah and You, RSC Adv. 2 (2012) 11169), anthraquinones (Comini et al., Phytomedicine. 18 (2011) 1093), xanthenes (Gianotti et al., Chem. - A Eur. J. 20 (2014) 10921), cyanines (Usama et al., ACS Appl. Bio Mater. 1 (2018) 1195), curcuminoids (Tonon et al., J. Contemp. Dent. Pract. 16 (2015) 1), phenothiazines and benzophenothiazines (Cincotta et al., Cancer Res. 54 (1994) 1249; Saucin and Van de Vorst, Radiat. Environ. Biophys. 17 (1980) 159). Besides the use of extended π conjugation, design of covalent dimerization based structures, introduction of TEMPO or heavy atom (chlorine, bromine, iodine) substitutions, chelating with metals are known to enhance photodynamic properties of the photosensitizers (Atchison et al., Chem. Commun. 53 (2017) 2009). Despite the numerous advancements and knowledge acquired in the field (Abrahamse and Hamblin, Biochem. J. 473 (2016) 347), development of photosensitizers suitable for PDT is a challenging task as these compounds need to meet many requirements, such as low dark toxicity but strong photocytotoxicity, long-wavelength absorbance peak, selectivity towards target cells, etc. (Huang, Technol. Cancer Res. Treat. 4 (2005) 283). Therefore, synthesis of new types of photosensitizers remains relevant in current organic chemistry.

US2016/213780 discloses compounds for PDT used in the treatment of bacterial infections. DOS SANTOS A FERREIRA ET AL ("Photodynamic therapy in cancer treatment - an update review", JOURNAL OF CANCER METASTASIS AND TREATMENT, vol. 2019, 29 March 2019) disclose compounds for PDT used for the treatment of cancer.

The present invention provides a series of novel substituted pyrazole-indole conjugated derivatives that are useful for photodynamic therapy. Hence, they can be used as anticancer photosensitizing drugs in treatments of skin and epithelial tumors accessible to light irradiation, or as antimicrobial photosensitizing agents.

### Disclosure of the invention

The object of the present invention are new conjugated pyrazole-indole derivatives, which possess photodynamic properties and low dark toxicity. These properties make them suitable to be used as anticancer and antibacterial agents and antibiotics.

The scope of the invention is defined by the claims. References to methods of treatment in the description of the invention are to be interpreted as references to the compounds, pharmaceutical compositions and medicaments of the present invention for use in a method for treatment of the human (or animal) body by therapy.

The pyrazole-indole derivatives of the present invention have a general formula I: 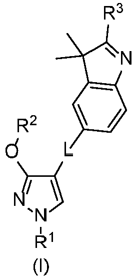 wherein:
L is a linker selected from -CH=CH-, -CH=CH-CH=CH- and -C≡C-;
R¹ is phenyl which can be unsubstituted or optionally substituted with 1 to 2 substituents selected from the group comprising F, methyl and methoxy.
R² is C1-C8 alkyl, preferably C1-C6 alkyl, which can be branched or linear, wherein in the alkyl chain one or two carbon atoms may optionally be replaced by one or two heteroatoms independently selected from the group comprising of O, N or S;
R³ is phenyl, which can be unsubstituted or optionally substituted with 1 to 2 substituents selected from the group comprising F, Cl, and methoxy.
and pharmaceutically acceptable salts thereof.

In a preferred embodiment, the present invention relates to compounds of formula I wherein L is -C≡C-, - CH=CH- or -CH=CH-CH=CH-, R¹ is phenyl; R² is hexyl; R³ is phenyl.

As used herein, the substituent groups have the following meanings:
methyl denotes the group -CH₃,
methoxy denotes the group -OCH₃,
alkyl denotes branched or unbranched alkyl chain containing 1 to 8 carbon atoms, preferably methyl, ethyl, propyl, isopropyl, butyl, isobutyl, pentyl, isopentyl, hexyl, isohexyl, heptyl, isoheptyl, or octyl. An example of an alkyl chain in which a carbon atom is replaced by a heteroatom can be, for example, 2-methoxyethyl.

An object of this invention are conjugated pyrazole-indole derivatives of the general formula I for use as medicaments.

Another object of this invention are conjugated pyrazole-indole derivatives of the general formula I for use as photosensitisers for inhibiting cell proliferation and/or inducing cell death.

An object of this invention are conjugated pyrazole-indole derivatives of the general formula I for use in the treatment of disorders selected from the group comprising cancer, skin diseases, inflammatory diseases and bacterial infections.

A further object of this invention are conjugated pyrazole-indole derivatives of the general formula I for use in the manufacture of a medicament for the treatment of diseases selected from the group consisting of psoriasis, acne, bacterial infections, basal cell carcinoma, squamous cell carcinoma, melanoma, cancer of lung, head, mouth, larynx, bladder, nasopharyngeal, vaginal and cervical cancers.

In addition to therapeutic applications, the compounds of the invention can be used as an agent for controlling cell proliferation *in vitro,* for instance, in cell cultures and/or tissue cultures.

Another object of this invention are conjugated pyrazole-indole derivatives of the general formula I as photosensitizing compounds in anticancer therapies.

An object of this invention are conjugated pyrazole-indole derivatives of the general formula I for use in the treatment of bacterial and microbial infections of skin, oral cavity, internal surfaces of the upper respiratory tract and internal surfaces of the genitourinary tract. Compounds of this invention have antimicrobial activity against at least Gram-positive bacteria.

After a pre-incubation with conjugated pyrazole-indole derivatives of the general formula I, light irradiation is applied to the treatment site. The preferred wavelength of the activating light irradiation is between 400 nm and 450 nm, preferably corresponding to the maximum absorption of the given compound of formula I. The irradiation can be performed using a non-coherent radiation such as from a diode-based source, or using a coherent radiation such as laser radiation. For surface treatments, a light source may be effective in irradiating specific target areas; for lesions within the body (inner cavities), an optical fiber apparatus or other devices may be used to irradiate target areas.

The invention further relates to a pharmaceutical composition, which comprises at least one conjugated pyrazole-indole derivative of the general formula I, and a pharmaceutically acceptable carrier.

The following derivatives of formula I are particularly preferred:
5-{[3-(Hexyloxy)-1-phenyl-1*H*-pyrazol-4-yl]ethynyl}-3,3-dimethyl-2-phenyl-3*H*-indole **(5)**
5-{(*E*)-2-[3-(Hexyloxy)-1-phenyl-1*H*-pyrazol-4-yl]ethenyl}-3,3-dimethyl-2-phenyl-3*H*-indole **(7)**
5-{(1*E*,3*E*)-4-[3-(Hexyloxy)-1-phenyl-1*H*-pyrazol-4-yl]buta-1,3-dien-1-yl}-3,3-dimethyl-2-phenyl-3*H-*indole **(10).**

### Pharmaceutical Compositions

The therapeutic composition comprises from 1% to 95% of the active ingredient, single-dose forms of administration preferably comprising from 20% to 90% of the active ingredient and administration forms, which are not single-dose preferably comprising from 5% to 20% of the active ingredient. Unit dose forms may be, for example, coated tablets, tablets, ampoules, vials, suppositories or capsules. Other forms of administration are, for example, ointments, creams, pastes, foams, tinctures, lipsticks, drops, sprays, dispersions and the like. Examples are capsules containing from about 0.05 g to about 1.0 g of the active ingredient.

The pharmaceutical compositions of the present invention are prepared in a manner known per se, for example by means of conventional mixing, granulating, coating, dissolving or lyophilizing processes.

Preferably, solutions of the active ingredient, and in addition also suspensions or dispersions, especially isotonic aqueous solutions, dispersions or suspensions, are used, if being possible for these to be prepared before use, for example in the case of lyophilized compositions which comprise the active substance by itself or together with a carrier, for example mannitol. The pharmaceutical compositions can be sterilized and/or comprise excipients, for example preservatives, stabilizers, wetting agents and/or emulsifiers, solubilizing agents, salts for regulating the osmotic pressure and/or buffers, and they are prepared in a manner known per se, for example by means of conventional dissolving or lyophilizing processes. The solutions or suspensions mentioned can comprise viscosity-increasing substances, such as sodium carboxymethylcellulose, dextran, polyvinylpyrrolidone or gelatine. Suspensions in oil comprise, as the oily component, the vegetable, synthetic or semi-synthetic oils customary for injection purposes. Oils which may be mentioned are, in particular, liquid fatty acid esters which contain, as the acid component, a long-chain fatty acid having 8 - 22, in particular 12-22, carbon atoms, for example lauric acid, tridecylic acid, myristic acid, pentadecylic acid, palmitic acid, margaric acid, stearic acid, arachidonic acid, behenic acid or corresponding unsaturated acids, for example oleic acid, elaidic acid, euric acid, brasidic acid or linoleic acid, if appropriate with the addition of antioxidants, for example vitamin E, β-carotene or 3,5-di-tert-butyl-4-hydroxytoluene. The alcohol component of these fatty acid esters has not more than 6 carbon atoms and is mono- or polyhydric, for example mono-, di- or trihydric alcohol, for example methanol, ethanol, propanol, butanol, or pentanol, or isomers thereof, but in particular glycol and glycerol. Fatty acid esters are, for example: ethyl oleate, isopropyl myristate, isopropyl palmitate, "Labrafil M 2375" (polyoxyethylene glycerol trioleate from Gattefoseé, Paris), "Labrafil M 1944 CS" (unsaturated polyglycolated glycerides prepared by an alcoholysis of apricot kernel oil and made up of glycerides and polyethylene glycol esters; from Gattefoseé, Paris), "Labrasol" (saturated polyglycolated glycerides prepared by an alcoholysis of TCM and made up of glycerides and polyethylene glycol esters; from Gattefose6, Paris) and/or "Miglyol 812" (triglyceride of saturated fatty acids of chain length C8 to C12 from Hüls AG, Germany), and in particular vegetable oils, such as cottonseed oil, almond oil, olive oil, castor oil, sesame oil, soybean oil and, in particular, groundnut oil.

The preparation of the injection compositions is carried out in the customary manner under sterile conditions, as are bottling, for example into ampoules or vials, and closing of the containers.

For example, pharmaceutical compositions for oral use can be obtained by combining the active ingredient with one or more solid carriers, if appropriate granulating the resulting mixture, and, if desired, processing the mixture or granules to tablets or coated tablet cores, if appropriate by addition of additional excipients. Suitable carriers are, in particular, fillers, such as sugars, for example lactose, sucrose, mannitol or sorbitol, cellulose preparations and/or calcium phosphates, for example tricalcium diphosphate, or calcium hydrogen phosphate, and furthermore binders, such as starches, for example maize, wheat, rice or potato starch, methylcellulose, hydroxypropylmethylcellulose, sodium carboxymethylcellulose and/or polyvinylpyrrolidine, and/or, if desired, desintegrators, such as the above mentioned starches, and furthermore carboxymethyl-starch, cross-linked polyvinylpyrrolidone, alginic acid or a salt thereof, such as sodium alginate. Additional excipients are, in particular, flow regulators and lubricants, for example salicylic acid, talc, stearic acid or salts thereof, such as magnesium stearate or calcium stearate, and/or polyethylene glycol, or derivatives thereof.

Coated tablet cores can be provided with suitable coatings which, if appropriate, are resistant to gastric juice, the coatings used being, inter alia, concentrated sugar solutions, which, if appropriate, comprise gum arabic, talc, polyvinylpyrrolidine, polyethylene glycol and/or titanium dioxide, coating solutions in suitable organic solvents or solvent mixtures or, for the preparation of coatings which are resistant to gastric juice, solutions of suitable cellulose preparations, such as acetylcellulose phthalate or hydroxypropylmethylcellulose phthalate. Dyes or pigments can be admixed to the tablets or coated tablet coatings, for example for identification or characterisation of different doses of active ingredient.

Pharmaceutical compositions, which can be used orally, are also hard capsules of gelatine and soft, closed capsules of gelatine and a plasticiser, such as glycerol or sorbitol. The hard capsules can contain the active ingredient in the form of granules, mixed for example with fillers, such as maize starch, binders and/or lubricants, such as talc or magnesium stearate, and stabilisers if appropriate. In soft capsules, the active ingredient is preferably dissolved or suspended in suitable liquid excipients, such as greasy oils, paraffin oil or liquid polyethylene glycol or fatty acid esters of ethylene glycol or propylene glycol, it being likewise possible to add stabilisers and detergents, for example of the polyethylene sorbitan fatty acid ester type.

Other oral forms of administration are, for example, syrups prepared in the customary manner, which comprise the active ingredient, for example, in suspended form and in a concentration from 5% to 20%, preferably around 10% or in a similar concentration which results in a suitable individual dose, for example, when 5 or 10 ml are measured out. Other forms are, for example, also pulverulent or liquid concentrates for preparing of shakes, for example in milk. Such concentrates can also be packed in unit dose quantities.

Pharmaceutical compositions, which can be used rectally, are, for example, suppositories that comprise a combination of the active ingredient with a suppository base. Suitable suppository bases are, for example, naturally occurring or synthetic triglycerides, paraffin hydrocarbons, polyethylene glycols or higher alkanols.

Compositions which are suitable for parental administration are aqueous solutions of an active ingredient in water-soluble form, for example of water-soluble salt, or aqueous injection suspensions, which comprise viscosity-increasing substances, for example sodium carboxymethylcellulose, sorbitol and/or dextran, and, if appropriate, stabilizers. The active ingredient can also be present here in the form of a lyophilisate, if appropriate, together with excipients, and be dissolved before parenteral administration by addition of suitable solvents. Solutions such as are used, for example, for parental administration can also be used as infusion solutions. Preferred preservatives are, for example, antioxidants, such as ascorbic acid, or microbicides, such as sorbic or benzoic acid.

Tinctures and solutions usually comprise an aqueous-ethanolic base to which, humectants for reducing evaporation, such as polyalcohols, for example glycerol, glycols and/or polyethylene glycol, and re-oiling substances, such as fatty acid esters with lower polyethylene glycols, i.e. lipophilic substances soluble in the aqueous mixture to substitute the fatty substances removed from the skin with ethanol, and, if necessary, other excipients and additives, are admixed.

The invention also relates to a process or method for treatment of the disease states mentioned above. The compounds can be administered therapeutically as such or in the form of pharmaceutical compositions, preferably in an amount, which is effective against the diseases mentioned. With a warm-blooded animal, for example a human, requiring such treatment, the compounds are used, in particular, in the form of pharmaceutical composition. A daily dose of about 0.1 to about 5 g, preferably 0.5 g to about 2 g, of a compound of the present invention may be administered for a body weight of about 70 kg.

### Brief Description of Drawings

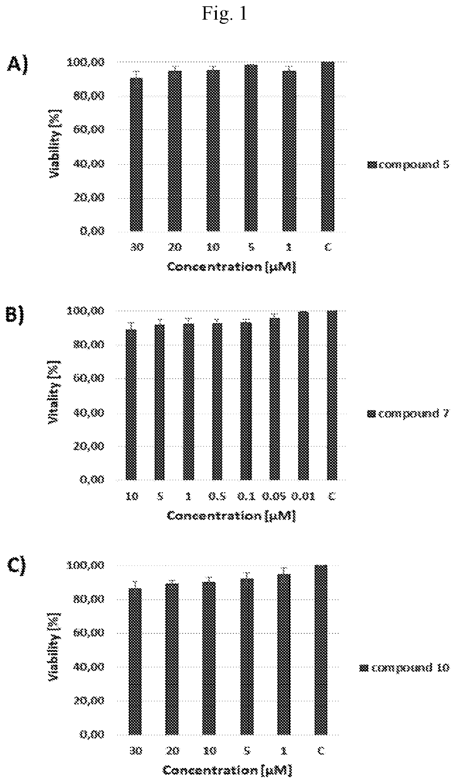

Fig. 1 displays dark viability of G361 cell line treated with compounds **5, 7** and **10.**
Fig. 2 shows production of reactive oxygen species in G361 cell line treated with compound **7** at 0.052 µM and irradiated with 50 J/cm² (414 nm).
Fig. 3 shows mitochondrial membrane potential changes in G361 cell line treated with compound **7** at 0.052 µM and irradiated with 50 J/cm² (414 nm).
Fig. 4 shows detection of DNA damage by a comet assay in G361 cells visualized using SYBR Green. **A** - untreated control cells; **B** - cells treated with compound **7** at 0.052 µM and irradiated with 50 J/cm² (414 nm).
Fig. 5 shows that treatment of G361 cells with a combination of compound **7** and light irradiation (414 nm) increases histone H2A.X phosphorylation at Ser-139.
Fig. 6 illustrates antibacterial effect of compound **7** in a combination with blue light irradiation (414 nm).

### Examples of carrying out the Invention

All chemicals and solvents were purchased from commercial suppliers and used without further purification unless otherwise specified. Prior to use, *N*,*N*-dimethylformamide was stored over molecular sieves (4 Å), while toluene and dichloromethane were dried over anhydrous sodium sulfate. All reactions were performed in oven dried flasks under argon atmosphere with magnetic stirring. Reaction progress was monitored by TLC analysis on Macherey-Nagel^{™} ALUGRAM^{®} Xtra SIL G/UV254 plates. TLC plates were visualized with UV light (wavelengths 254 and 365 nm) or iodine vapour. Compounds were purified by flash chromatography in a glass column (stationary phase - silicagel, high-purity grade 9385, pore size 60 Å, particle size - 230-400 mesh, supplier Sigma-Aldrich). ¹H and ¹³C NMR spectra were recorded at room temperature on a Bruker Avance III 700 spectrometer (700 MHz for ¹H and 176 MHz for ¹³C) in CDCl₃ as solvent. FT-IR spectra were collected using ATR method on a Bruker Vertex 70v spectrometer with an integrated Platinum ATR accessory. The melting points of crystalline compounds were determined in open capillary tubes with a Buchi M-565 apparatus (temperature gradient - 2 °C/min) and are uncorrected. High-resolution mass spectrometry (HRMS) spectra were obtained in ESI mode on a Bruker MicrOTOF-Q III spectrometer. UV-Vis absorption and fluorescence spectra were registered on an Infinity M200 Pro Tecan microplate reader.

Compounds of formula I are obtainable according to general schemes **1-3** given below:

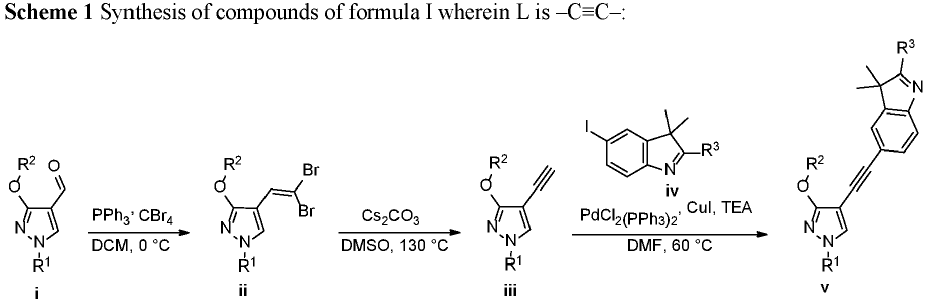

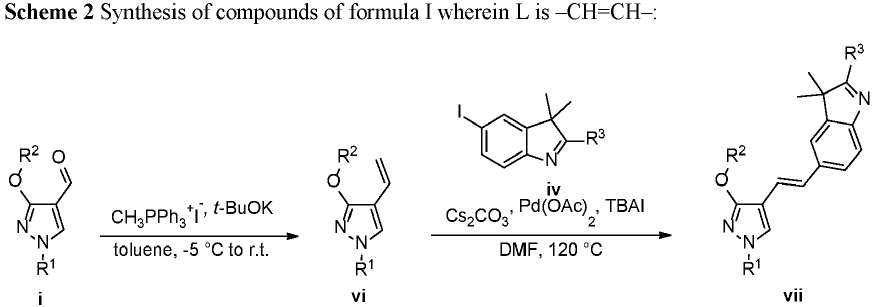

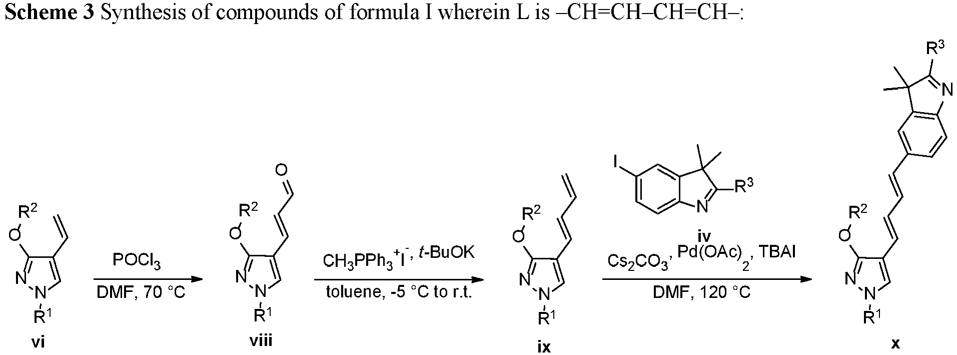

### Synthetic scheme of target compound 5-{[3-(hexyloxy)-1-phenyl-1H-pyrazol-4-yl]ethynyl}-3,3-dimethyl-2-phenyl-3H-indole 5 (Examples 1-3):

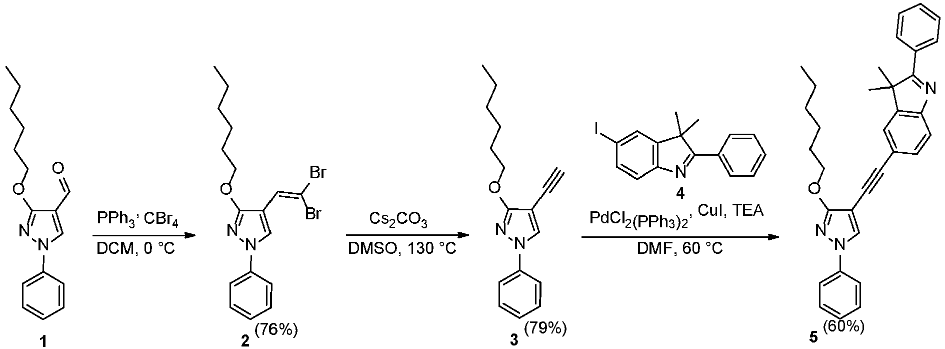

### Example 1 4-(2,2-Dibromoethenyl)-3-(hexyloxy)-1-phenyl-1H-pyrazole (intermediate compound 2)

Tetrabromomethane (6633 mg, 20 mmol) was dissolved in dry dichloromethane (25 mL) at -10 °C and a solution of triphenylphosphine (10492 mg, 40 mmol) in dry dichloromethane (25 mL) was added dropwise. The reaction mixture was stirred at -10 °C for 45-60 min until an orange solution was obtained. Thereafter, aldehyde **1** (prepared as described previously by Kazlauskas et al., J. Phys. Chem. C. 118 (2014) 25261-25271) was dissolved in dry dichloromethane (25 mL) and added dropwise to the mixture. After warming up to room temperature, the reaction was carried out for 2 h, and the progress was monitored by TLC. Upon completion, the reaction was quenched by water (50 × mL) and the organic layer was separated, dried over anhydrous Na₂SO₄, filtered, and concentrated under reduced pressure. The residue was purified by column chromatography on silicagel (n-hexane/ethylacetate 15:1 v/v) to afford compound **2** as a colorless liquid (3264 mg, 76%). R*_{f}* = 0.54 (n-hexane/ethylacetate 15:1 v/v). ¹H NMR (700 MHz, CDCl₃, ppm): δ 8.44 (s, 1H), 7.62 (d, *J* = 7.8 Hz, 2H), 7.42 (t, *J* = 7.9 Hz, 2H), 7.29 (s, 1H), 7.24 (t*, J* = 7.4 Hz, 1H), 4.31 (t, *J* = 6.7 Hz, 2H), 1.81 (p, *J=* 6.8 Hz, 2H), 1.46 (p, *J=* 7.2 Hz, 2H), 1.39 - 1.32 (m, 4H), 0.92 (t, *J=* 6.9 Hz, 3H). ¹³C NMR (176 MHz, CDCl₃, ppm): δ 162.1, 139.8, 129.4, 125.94, 125.82, 125.50, 118.1, 105.7, 85.4, 69.4, 31.6, 29.1, 25.6, 22.6, 14.1. ¹⁵N NMR (71 MHz, CDCl₃, ppm): δ -123.1 (N-2), -185.7 (N-1). IR (neat, vₘₐₓ, cm⁻¹): 3070, 3050, 3026 (CH_{aromatic,vinyl}), 2952, 2928, 2869, 2857 (CH_{aliphatic}), 1599, 1562, 1500, 1465, 1415, 1374, 1296, 1222, 1177, 1057 (C=C, C=N, CH₂/CH_{3 bending}, C-N, C-O), 1000, 942, 882, 804, 751, 685, 670 (CH_{aromatic, vinyl out-of-plane bending}). HRMS (ESI-TOF): for C₁₇H₂₀Br₂N₂NaO ([M+Na]⁺): calcd. 448.9835; found 448.9838.

### Example 2 4-Ethynyl-3-(hexyloxy)-1-phenyl-1H-pyrazole (intermediate compound 3)

4-(2,2-Dibromoethenyl)-3-(hexyloxy)-1-phenyl-1*H-*pyrazole **2** (2141 mg, 5 mmol) was dissolved in dimethylsulfoxide (10 mL) and caesium carbonate (4887 mg, 15 mmol) was added into the solution. The reaction mixture was stirred at 130 °C for 12 h. Upon completion, the reaction mixture was poured into saturated sodium chloride solution (50 mL) and extracted with EtOAc (4 × 25 mL). The organic layer was dried over anhydrous Na₂SO₄, filtered, and concentrated under reduced pressure. The residue was purified by column chromatography on silica gel (n-hexane/ethylacetate 15:1 v/v) to afford compound 3 as an orange liquid (1057 mg, 79%). R*_{f}* = 0.40 (n-hexane/ethylacetate 15:1 v/v). ¹H NMR (700 MHz, CDCl₃, ppm): δ 7.89 (s, 1H), 7.57 (d, *J=* 7.9 Hz, 2H), 7.41 (t, *J=* 8.0 Hz, 2H), 7.23 (t, *J=* 7.4 Hz, 1H), 4.35 (t, *J* = 6.8 Hz, 2H), 3.19 (s, 1H), 1.85 (p, *J=* 6.9 Hz, 2H), 1.48 (p, *J=* 7.2 Hz, 2H), 1.39 - 1.32 (m, 4H), 0.92 (t, *J* = 7.0 Hz, 3H). ¹³C NMR (176 MHz, CDCl₃, ppm): δ 164.6, 139.6, 130.6, 129.5, 126.0, 118.2, 91.2, 80.7, 73.7, 69.9, 31.7, 29.1, 25.7, 22.7, 14.2. ¹⁵N NMR (71 MHz, CDCl₃, ppm): δ -120.6 (N-2), -187.8 (N-1). IR (neat, vₘₐₓ,cm⁻¹): 3289 (≡C-H), 3110, 3071, 3051 (CH_{aromatic}), 2953, 2930, 2870, 2858 (CH_{aliphatic}), 2117 (C≡C), 1599, 1565, 1503, 1466, 1409, 1363, 1216, 1198, 1054 (C=C, C=N, CH₂/CH_{3 bending}, C-N, C-O), 998, 941, 901, 810, 753, 688, 677 (CH_{aromatic out-of-plane bending}). HRMS (ESI-TOF): for C₁₇H₂₀N₂NaO ([M+Na]+): calcd. 291.1468; found 291.1469.

### Example 3 5-{[3-(Hexyloxy)-1-phenyl-1H-pyrazol-4-yl]ethynyl}-3,3-dimethyl-2-phenyl-3H-indole (target compound 5)

Under an atmosphere of argon, to a solution of 4-ethynyl-3-(hexyloxy)-1-phenyl-1*H*-pyrazole **3** (313 mg, 1.16 mmol) and 5-iodo-3,3-dimethyl-2-phenyl-3*H*-indole **4** (prepared as described by M. Tomasulo et al., J. Org. Chem. 73 (2008) 118-126) (405 mg, 1.16 mmol), in dry dimethylformamide (5 mL), copper(I) iodide (442 mg, 2.32 mmol), triethylamine (0.3 mL, 2.32 mmol) and tetrakis(triphenylphosphine)palladium(0) (81.4 mg, 0.116 mmol) were added. The reaction was stirred at 60 °C for 24 h. Upon completion, the reaction mixture was cooled down to room temperature, diluted with distilled water (50 mL) and extracted with EtOAc (4 × 25 mL). The combined organic extracts were dried over anhydrous Na₂SO₄, filtered and concentrated under reduced pressure. The residue was purified by column chromatography on silica gel (n-hexane/ethylacetate 9:1 v/v) to yield the compound **5** as a brown-orange sticky solid (339 mg, 60%). R*_{f}* = 0.38 (n-hexane/ethylacetate 6:1 v/v). ¹H NMR (700 MHz, CDCl₃, ppm): δ 8.16 (dd, *J=* 2.8, 6.6 Hz, 2H), 7.95 (s, 1H), 7.66 (d, *J=* 7.9 Hz, 1H), 7.62 (d, *J=* 8.3 Hz, 2H), 7.54 (d, *J=* 8.0 Hz, 1H), 7.51 (d, *J=* 6.3 Hz, 2H), 7.49 (d, *J=* 2.5 Hz, 2H), 7.43 (t, *J=* 7.8 Hz, 2H), 7.23 (t, *J* = 7.4 Hz, 1H), 4.40 (t, *J=* 6.8 Hz, 2H), 1.90 (p, *J=* 6.9 Hz, 2H), 1.61 (s, 6H), 1.53 (p, *J=* 7.3 Hz, 2H), 1.43 - 1.34 (m, 4H), 0.93 (t, J= 6.9 Hz, 3H). ¹³C NMR (176 MHz, CDCl₃, ppm): δ 184.1, 164.2, 153.0, 147.8, 139.7, 133.2, 131.6, 130.9, 129.67, 129.52, 128.75, 128.46, 125.9, 124.2, 120.97, 120.86, 118.0, 93.1, 92.6, 79.4, 69.9, 53.7, 31.7, 29.2, 25.7, 24.8, 22.7, 14.2. ¹⁵N NMR (71 MHz, CDCl₃, ppm): δ -70.7 (indole N-1), -120.5 (pyrazole N-2), -187.8 (pyrazole N-1). IR (neat, vₘₐₓ,cm⁻¹): 3110, 3053 (CH_{aromatic}), 2954, 2928, 2869, 2857 (CH_{aliphatic}), 2215 (C=C), 1599, 1570, 1511, 1460, 1410, 1363, 1296, 1214, 1141, 1054 (C=C, C=N, CH₂/CH_{3 bending}, C-N, C-O), 990, 940, 999, 830, 753, 690, 676 (CH_{aromatic out-of-plane bending}). HRMS (ESI-TOF): for C₃₃H₃₄N₃O ([M+H]⁺): calcd. 488.2696; found 488.2700.

### Synthetic scheme of target compound 5-{(E)-2-[3-(hexyloxy)-1-phenyl-1H-pyrazol-4-yl]ethenyl}-3,3-dimethyl-2-phenyl-3H-indole 7 (examples 4-5):

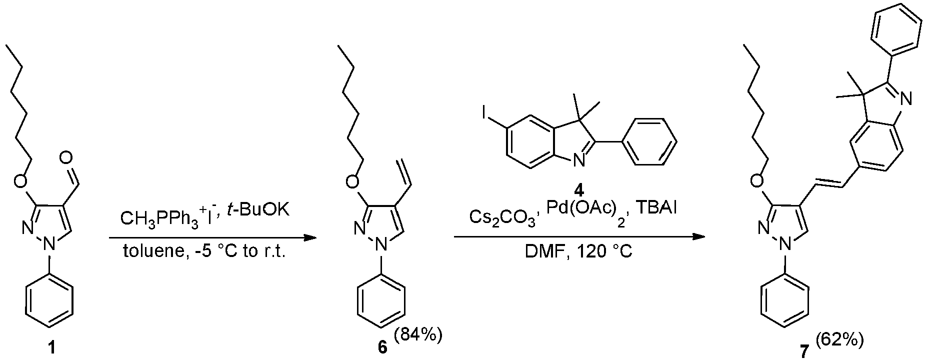

### Example 4 4-Ethenyl-3-(hexyloxy)-1-phenyl-1H-pyrazole (intermediate compound 6)

To a suspension of methyltriphenylphosphonium iodide (1.83 g, 4.5 mmol) in dry toluene (60 mL), under inert atmosphere potassium tert-butoxide (1.01 g, 9 mmol) was added in one portion. The reaction mixture was stirred at room temperature for 30 min and subsequently refluxed for another 30 min. Formation of the ylide can be visually observed by its persistent yellow color. After refluxing, the reaction mixture was allowed to cool down to room temperature and was placed in an ice bath, followed by dropwise addition of an aldehyde **1** (prepared as described by Kazlauskas et al., J. Phys. Chem. C. 118 (2014) 25261-25271) (817 mg, 3 mmol) dissolved in dry toluene (30 mL). The reaction was carried out at room temperature for 3 h, and the progress was monitored by TLC. Upon completion, the reaction was quenched by saturated solution of ammonium chloride (90 × mL) and the organic layer was separated, dried over anhydrous Na₂SO₄, filtered, and concentrated under reduced pressure. The residue was purified by column chromatography on silicagel (n-hexane/ethylacetate 10:1 v/v) to afford compound **6** as a yellow liquid (681 mg, 84%). R*_{f}* = 0.74 (n-hexane/ethylacetate 6:1 v/v). ¹H NMR (700 MHz, CDCl₃, ppm): δ 7.73 (s, 1H), 7.58 (d, *J* = 8.0 Hz, 2H), 7.40 (t, *J* = 7.9 Hz, 2H), 7.19 (t, *J* = 7.3 Hz, 1H), 6.54 (dd, *J* = 11.3, 17.7 Hz, 1H), 5.73 (dd, *J* = 1.6, 17.7 Hz, 1H), 5.16 (dd, *J=* 1.6, 11.3 Hz, 1H), 4.35 (t, *J=* 6.6 Hz, 2H), 1.84 (p, *J* = 6.7 Hz, 2H), 1.51 (p, *J=* 7.3 Hz, 2H), 1.41 - 1.35 (m, 4H), 0.93 (t, *J=* 7.0 Hz, 3H). ¹³C NMR (176 MHz, CDCl₃, ppm): δ 162.3, 140.1, 129.5, 125.29, 125.22, 125.17, 117.7, 113.2, 108.7, 69.2, 31.7, 29.3, 25.9, 22.7, 14.2. ¹⁵N NMR (71 MHz, CDCl₃, ppm): δ -119.9 (N-2), -189.7 (N-1). IR (neat, vₘₐₓ, cm⁻¹): 3100, 3071, 3051, 3015 (CH_{aromatic, vinyl}), 2954, 2930, 2870,2858 (CH_{aliphatic}), 1638, 1600, 1565, 1501, 1466, 1427, 1373, 1245, 1205, 1181, 1057, 1032 (C=C, C=N, CH₂/CH_{3 bending}, C-N, C-O), 990, 940, 897, 807, 751, 688, 659 (CH_{aromatic}, _{vinyl out-of-plane bending}). HRMS (ESI-TOF): for C₁₇H₂₂N₂NaO ([M+Na]⁺): calcd. 293.1624; found 293.1625.

### Example 5 5-{(E)-2-[3-(Hexyloxy)-1-phenyl-1H-pyrazol-4-yl]ethenyl}-3,3-dimethyl-2-phenyl-3H-indole (target compound 7)

4-Ethenyl-3-(hexyloxy)-1-phenyl-1*H*-pyrazole **6** (270 mg, 1 mmol), 5-iodo-3,3-dimethyl-2-phenyl-3H-indole **4** (prepared as described by M. Tomasulo et al., J. Org. Chem. 73 (2008) 118-126) (413 mg, 1.19 mmol), caesium carbonate (489 mg, 1.5 mmol) and tetrabutylammonium iodide (554 mg, 1.5 mmol) were dissolved in dry dimethylformamide (6 mL) under an atmosphere of argon. Subsequently, palladium(II) acetate (45 mg, 0.2 mmol) was added to the solution in one portion. The reaction mixture was stirred at 120 °C for 48 h. Upon completion, the reaction mixture was cooled down to room temperature, diluted with distilled water (50 mL) and extracted with EtOAc (4 × 25 mL). The combined organic extracts were dried over anhydrous Na₂SO₄, filtered and concentrated under reduced pressure. The residue was purified by column chromatography on silica gel (n-hexane/ethylacetate 12:1 v/v) to afford compound **7** as a bright yellow glassy solid (302 mg, 62% yield). R*_{f}* = 0.33 (n-hexane/ethylacetate 6:1 v/v). ¹H NMR (700 MHz, CDCl₃, ppm): δ 8.20 (dd, *J* = 1.5, 8.0 Hz, 2H), 7.85 (s, 1H), 7.71 (d, *J* = 8.4 Hz, 1H), 7.64 (d, *J* = 7.7 Hz, 2H), 7.53 - 7.47 (m, 5H), 7.42 (t, *J* = 7.9 Hz, 2H), 7.28 (d, *J* = 16.3 Hz, 1H), 7.20 (t, *J* = 7.4 Hz, 1H), 7.01 (d, *J=* 16.3 Hz, 1H), 4.46 (t, *J* = 6.7 Hz, 2H), 1.95 (p, *J* = 6.7 Hz, 2H), 1.65 (s, 6H), 1.60 (p, *J* = 7.4 Hz, 2H), 1.49 - 1.40 (m, 4H), 0.99 (t, *J=* 7.1 Hz, 3H). ¹³C NMR (176 MHz, CDCl₃, ppm): δ 182.8, 162.2, 152.5, 148.3, 139.9, 136.3, 133.4, 130.5, 129.3, 128.61, 128.26, 127.85, 126.0, 125.11, 124.87, 121.0, 118.2, 117.5, 116.9, 108.6, 69.2, 53.4, 31.6, 29.2, 25.8, 24.9, 22.7, 14.1. ¹⁵N NMR(71 MHz, CDCl₃, ppm): δ -70.0 (indole N-1), -119.3 (pyrazole N-2), -188.9 (pyrazole N-1). IR (neat, νₘₐₓ, cm⁻¹): 3105, 3050, 3005 (CH_{aromatic}, _{vinyl}), 2955, 2928, 2858 (CH_{aliphatic}), 1677, 1638, 1598, 1565, 1501, 1463, 1406, 1376, 1262, 1199, 1055 (C=C, C=N, CH₂/CH_{3 bending}, C-N, C-O), 989, 963, 940, 890, 821, 752, 690, 676 (CH_{aromatic}, _{vinyl out-of-plane bending}). HRMS (ESI-TOF)_{:} for C₃₃H₃₆N₃O ([M+H]⁺): calcd. 490.2853; found 490.2854_{.}

### Synthetic scheme of target compound 5-{(1E,3E)-4-[3-(hexyloxy)-1-phenyl-1H-pyrazol-4-yl]buta-1,3-dien-1-yl}-3,3-dimethyl-2-phenyl-3H indole 10 (examples 6-8):

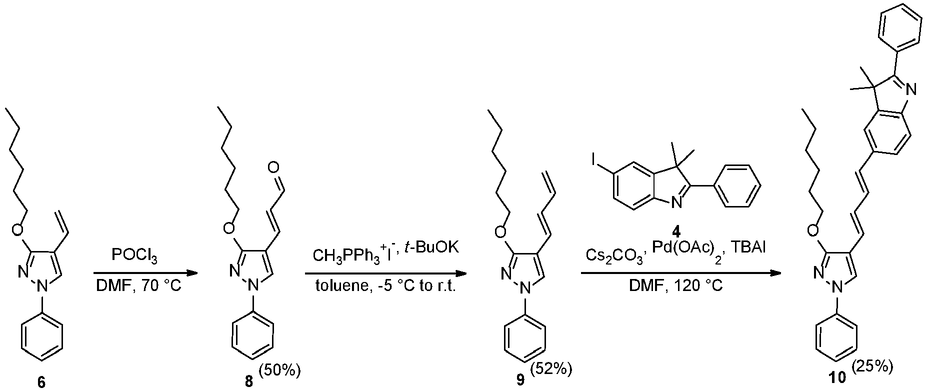

### Example 6 (2E)-3-[3-(Hexyloxy)-1-phenyl-1H-pyrazol-4-yl]prop-2-enal (intermediate compound 8)

Phosphorus oxychloride (1.86 mL, 20 mmol) was added dropwise to dry DMF (1.55 mL, 20 mmol) at -10 °C and the resulting mixture was stirred at the same temperature for 10-20 min until Vilsmeier-Haack complex formed. Subsequently, a solution of 4-ethenyl-3-(hexyloxy)-1-phenyl-1*H*-pyrazole **6** (1351 mg, 5 mmol) in dry DMF (15 mL) was added to the Vilsmeier-Haack complex and the temperature was slowly raised to 70 °C and maintained for 12 h. The reaction mixture was cooled in an ice bath, cautiously quenched with ice-cold water (100 mL), and basified with 10% NaHCO₃ solution. The precipitate was filtered off. Filtrate was extracted with ethyl acetate (4 × 50 mL) which was washed with brine, dried over anhydrous Na₂SO₄, filtered and concentrated under reduced pressure. The residue was purified by column chromatography on silicagel (n-hexane/ethylacetate 4:1 v/v) to yield the compound **8** as an orange crystalline solid (746 mg, 50%). M.p. 69.2 - 70.2 °C. R*_{f}* = 0.21 (n-hexane/ethylacetate 6:1 v/v).¹H NMR (700 MHz, CDCl₃, ppm): δ 9.57 (d, *J=* 8.0 Hz, 1H), 7.95 (s, 1H), 7.63 - 7.60 (m, 2H), 7.46 - 7.42 (m, 2H), 7.33 (d, *J=* 15.7 Hz, 1H), 7.28 - 7.25 (m, 1H), 6.72 (dd, *J=* 8.0, 15.7 Hz, 1H), 4.37 (t, *J=* 6.7 Hz, 2H), 1.84 (p, *J=* 6.7 Hz, 2H), 1.48 (p, *J=* 7.4 Hz, 2H), 1.39 - 1.32 (m, 4H), 0.91 (t, *J=* 7.1 Hz, 3H). ¹³C NMR (176 MHz, CDCl₃, ppm): δ 194.0, 162.8, 141.9, 139.3, 129.5, 128.1, 127.2, 126.4, 118.2, 106.5, 69.7, 31.5, 29.0, 25.7, 22.6, 14.0. ¹⁵N NMR (71 MHz, CDCl₃, ppm): δ -118.0 (N-2), -182.5 (N-1). IR (neat, vₘₐₓ, cm⁻¹): 3129, 3103, 3067, 3035 (CH_{aromatic}, _{vinyl}), 2999, 2954, 2929, 2861, 2815, 2735 (CH_{aliphatic}), 1668 (C=O), 1616, 1598, 1558, 1502, 1473, 1425, 1375, 1296, 1237, 1173, 1125, 1057, 1011 (C=C, C=N, CH₂/CH_{3 bending}, C-N, C-O), 979, 962, 937, 911, 836, 753, 713, 687, 675 (CH_{aromatic}, _{vinyl out-of-plane bending}). HRMS (ESI-TOF): for C₁₈H₂₂N₂NaO₂ ([M+Na]⁺): calcd. 321.1573; found 321.1573.

### Example 7 4-[(1E)-Buta-1,3-dien-1-yl]-3-(hexyloxy)-1-phenyl-1H-pyrazole (intermediate compound 9)

To a suspension of methyltriphenylphosphonium iodide (1.83 g, 4.5 mmol) in dry toluene (60 mL), under inert atmosphere potassium tert-butoxide (1.01 g, 9 mmol) was added in one portion. The reaction mixture was stirred at room temperature for 30 min and subsequently refluxed for another 30 min. Formation of the ylide can be visually observed by its persistent yellow color. After refluxing, the reaction mixture was allowed to cool down to room temperature and was placed in an ice bath, followed by dropwise addition of an aldehyde **8** (895 mg, 3 mmol) dissolved in dry toluene (30 mL). The reaction was carried out at room temperature for 2 h, and the progress was monitored by TLC. Upon completion, the reaction was quenched by saturated solution of ammonium chloride (90 × mL) and the organic layer was separated, dried over anhydrous Na₂SO₄, filtered, and concentrated under reduced pressure. The residue was purified by flash chromatography on silicagel (n-hexane/ethylacetate 9:1 v/v) to afford compound **9** as a yellowish liquid (462 mg, 52%). R*_{f}* = 0.65 (n-hexane/ethylacetate 9:1 v/v). ¹H NMR (700 MHz, CDCl₃, ppm): δ 7.70 (s, 1H), 7.57 - 7.54 (m, 2H), 7.39 - 7.36 (m, 2H), 7.18 - 7.14 (m, 1H), 6.79 (dd, *J* = 15.7, 10.6 Hz, 1H), 6.47 - 6.40 (m, 1H), 6.37 (d, *J* = 15.7 Hz, 1H), 5.27 - 5.19 (m, 1H), 5.08 - 5.03 (m, 1H), 4.34 (t, *J* = 6.6 Hz, 2H), 1.84 (p, *J=* 6.7 Hz, 2H), 1.50 (p, *J* = 7.4 Hz, 2H), 1.40 - 1.32 (m, 4H), 0.92 (t, *J=* 7.1 Hz, 3H). ¹³C NMR (176 MHz, CDCl₃, ppm): δ 162.1, 139.9, 137.9, 129.3, 129.0, 125.1, 124.9, 121.3, 117.5, 115.5, 108.2, 69.1, 31.6, 29.2, 25.8, 22.6, 14.1. ¹⁵N NMR (71 MHz, CDCl₃, ppm): δ -119.7 (N-2), -189.0 (N-1). IR (neat, vₘₐₓ,cm⁻¹): 3121, 3080, 3046 (CH_{aromatic}, _{vinyl}), 2999, 2947, 2933, 2870, 2859 (CH_{aliphatic}), 1632, 1597, 1553, 1501, 1464, 1400, 1362, 1266, 1246, 1199, 1154, 1054, 1006 (C=C, C=N, CH₂/CH_{3 bending}, C-N, C-O), 954, 905, 890, 807, 756, 692, 676 (CH_{aromatic}, _{vinyl out-of-plane bending}). HRMS (ESI-TOF): for C₁₉H₂₄N₂NaO ([M+Na]⁺): calcd. 319.1781; found 319.1781.

### Example 8 5-{(1E, 3E)-4-[3-(Hexyloxy)-1-phenyl-1H-pyrazol-4-yl]buta-1,3-dien-1-yl}-3,3-dimethyl-2-phenyl-3H-indole (target compound 10)

4-[(1*E*)-Buta-1,3-dien-1-yl]-3-(hexyloxy)-1-phenyl-1*H*-pyrazole **9** (362 mg, 1.22 mmol), 5-iodo-3,3-dimethyl-2-phenyl-3*H*-indole **4** (453 mg, 1.3 mmol), caesium carbonate (596 mg, 1.83 mmol) and tetrabutylammonium iodide (676 mg, 1.83 mmol) were dissolved in dry dimethylformamide (5 mL) under an atmosphere of argon. Subsequently, palladium(II) acetate (55 mg, 0.24 mmol) was added. The reaction was stirred at 120 °C for 48 h. Upon completion, the reaction was cooled down to room temperature, poured into saturated NaCl solution (50 mL) and extracted with EtOAc (4 × 25 mL). The combined organic extracts were dried over anhydrous Na₂SO₄, filtered and concentrated under reduced pressure. The residue was purified by column chromatography on silicagel (n-hexane/ethylacetate 6: 1 v/v) to yield compound **10** as a yellow amorphous solid (156 mg, 25%). R*_{f}* = 0.33 (n-hexane/ethylacetate 6:1 v/v). ¹H NMR (700 MHz, CDCl₃, ppm): δ 8.14 (dd, *J* = 1.7, 7.8 Hz, 2H), 7.71 (s, 1H), 7.63 (d, *J* = 7.9 Hz, 1H), 7.56 (d, *J* = 7.6 Hz, 2H), 7.47 - 7.42 (m, 3H), 7.41 - 7.38 (m, 2H), 7.36 (t, *J=* 7.9 Hz, 2H), 7.14 (t, *J=* 7.4 Hz, 1H), 6.98 (dd, *J=* 10.8, 15.1 Hz, 1H), 6.92 (dd, *J* = 10.8, 15.1 Hz, 1H), 6.64 (d, *J* = 15.1 Hz, 1H), 6.49 (d, *J* = 15.1 Hz, 1H), 4.37 (t, *J* = 6.6 Hz, 2H), 1.87 (p, *J* = 6.7 Hz, 2H), 1.59 (s, 6H), 1.53 (p, *J* = 7.4 Hz, 2H), 1.43 - 1.35 (m, 4H), 0.94 (t, J= 7.1 Hz, 3H). ¹³C NMR (176 MHz, CDCl₃, ppm): δ 182.9, 162.1, 152.6, 148.2, 139.8, 135.8, 133.3, 130.88, 130.45, 129.82, 129.28, 128.64, 128.57, 128.22, 126.4, 125.06, 124.70, 121.3, 121.0, 118.3, 117.4, 108.7, 69.1, 53.3, 31.6, 29.2, 25.7, 24.8, 22.6, 14.1. ¹⁵N NMR (71 MHz, CDCl₃, ppm): δ_{N} - 70.4 (indole N-1), -119.8 (pyrazole N-2), -188.7 (pyrazole N-1). IR (neat, νₘₐₓ, cm⁻¹): 3068, 3047, 3002 (CH_{aromatic, vinyl}), 2957, 2927, 2870, 2856 (CH_{aliphatic}), 1624, 1596, 1583, 1553, 1499, 1462, 1405, 1377, 1245, 1211, 1189, 1054 (C=C, C=N, CH₂/CH_{3 bending}, C-N, C-O), 993, 938, 993, 844, 796, 750, 688, 677 (CH_{aromatic}, _{vinyl out-of-plane bending}). HRMS (ESI-TOF): for C₃₅H₃₈N₃O ([M+H]⁺): calcd. 516.3009; found 516.3008.

**Table 1: Compounds prepared by the method of examples 1-8**

| No. | Substituent | | | | HRMS analysis | |
|---|---|---|---|---|---|---|
| | R¹ | R² | R³ | L | Calculated | Found |
| 5 | phenyl | hexyl | phenyl | -C≡C- | 488.2696 | 488.2700 |
| 7 | phenyl | hexyl | phenyl | -CH-CH- | 490.2853 | 490.2854 |
| 10 | phenyl | hexyl | phenyl | -CH=CH-CH=CH- | 516.3009 | 516.3008 |
| 11 | phenyl | pentyl | phenyl | -CH-CH- | 476.2696 | 476.2695 |
| 12 | phenyl | butyl | phenyl | -CH-CH- | 462.2540 | 462.2541 |
| 13 | phenyl | propyl | phenyl | -CH-CH- | 448.2383 | 448.2383 |
| 14 | phenyl | ethyl | phenyl | -CH-CH- | 434.2227 | 434.2228 |
| 15 | phenyl | methyl | phenyl | -CH-CH- | 420.2070 | 420.2070 |
| 16 | phenyl | 2-methoxyethyl | phenyl | -CH-CH- | 464.2333 | 464.2334 |
| 17 | 4-methoxyphenyl | hexyl | phenyl | -CH-CH- | 520.2959 | 520.2959 |
| 18 | 4-methoxyphenyl | methyl | phenyl | -CH-CH- | 450.2176 | 450.2176 |
| 19 | 4-methoxyphenyl | 2-methoxyethyl | phenyl | -CH-CH- | 494.2438 | 494.2437 |
| 20 | 4-fluorophenyl | hexyl | phenyl | -CH-CH- | 508.2759 | 508.2759 |
| 21 | 4-fluorophenyl | methyl | phenyl | -CH-CH- | 438.1976 | 438.1977 |
| 22 | 4-fluorophenyl | 2-methoxyethyl | phenyl | -CH-CH- | 482.2238 | 482.2240 |
| 23 | 4-methoxyphenyl | hexyl | phenyl | -CH-CH- | 504.3009 | 504.3009 |
| 24 | 4-methoxyphenyl | methyl | phenyl | -CH-CH- | 434.2227 | 434.2229 |
| 25 | 4-methoxyphenyl | 2-methoxyethyl | phenyl | -CH-CH- | 478.2489 | 478.2489 |
| 26 | phenyl | hexyl | 4-fluorophenyl | -CH-CH- | 508.2759 | 508.2757 |
| 27 | phenyl | methyl | 4-fluorophenyl | -CH-CH- | 438.1976 | 438.1977 |
| 28 | phenyl | 2-methoxyethyl | 4-fluorophenyl | -CH-CH- | 482.2238 | 482.2236 |
| 29 | phenyl | hexyl | 4-chlorophenyl | -CH-CH- | 524.2463 | 524.2463 |
| 30 | phenyl | methyl | 4-chlorophenyl | -CH-CH- | 454.1681 | 454.1681 |
| 31 | phenyl | 2-methoxyethyl | 4-chlorophenyl | -CH-CH- | 498.1943 | 498.1943 |
| 32 | phenyl | hexyl | 3,4-dimethoxyphenyl | -CH-CH- | 550.3064 | 550.3064 |
| 33 | phenyl | methyl | 3,4-dimethoxyphenyl | -CH-CH- | 480.2282 | 480.2282 |
| 34 | phenyl | 2-methoxyethyl | 3,4-dimethoxyphenyl | -CH-CH- | 524.2544 | 524.2542 |
| 35 | phenyl | hexyl | 4,6-dimethoxyphenyl | -CH-CH- | 550.3064 | 550.3064 |
| 36 | phenyl | methyl | 4,6-dimethoxyphenyl | -CH-CH- | 480.2282 | 480.2282 |
| 37 | phenyl | 2-methoxyethyl | 4,6-dimethoxyphenyl | -CH-CH- | 524.2544 | 524.2544 |

### Example 9 Fluorescence properties

Fluorescence properties of final compounds **5, 7** and **10** were investigated in water. The excitation wavelength *λ*ₑₓ was set to 360 nm for compound **5** and to 385 nm for compounds **7** and **10.** The emission maxima *λ*ₑₘ of all three compounds are located in the 475-525 nm range, which corresponds to the blue part of the visible light spectrum.

Replacing ethynyl bridge in derivative **5** with ethenyl in compound **7,** resulted in a bathochromical shift of the emission maxima. On the other hand, as expected an additional double bond in compound **10,** compared to compound **7,** caused both a bathochromic shift of the emission maxima. Notably, Stokes shifts of all the compounds **5, 7** and **10** are at least 115 nm, reaching as high as 145 nm for compound **10** (Table 2).

**Table 2. Absorption and fluorescence characteristics of compounds 5, 7 and 10 in water.**

| **Compound** | **λ_{abs}**, **nm** | **λₑₘ**, **nm** | **Stokes shift, nm** |
|---|---|---|---|
| **5** | 355 | 475 | 120 |
| **7** | 380 | 495 | 115 |
| **10** | 380 | 525 | 145 |

### Example 10 Cell culture and cell viability assay

G361 cell line (human skin melanoma) was cultivated in DMEM medium supplemented with 10% fetal bovine serum, 1% penicillin/streptomycin, 1% glutamine, at 37 °C in 5% CO₂ atmosphere. Viability of cells was determined using the MTT (Sigma-Aldrich) assay in 96-well microplates (10⁴ per well in 100 µl of DMEM). The test compounds were added 24 h post plating and the cells were then incubated for additional 24 h. Cells were then exposed to an irradiation in 100 µl of PBS with 5 mM glucose and incubated for further 24 h. After the experiment, 50 µl of MTT (dissolved in PBS) was added and cells were incubated for another 3 h at 37 °C and in 5 % CO₂. The media was then carefully replaced with 100 µl of DMSO in order to solubilize the violet formazan crystals. The measurement of absorbance was carried out on reader Synergy HT at 570 nm and 690 nm. The cell viability of the samples was determined as a percentage of the control cell viability (100 × average of test group / average of control group). The data for the determination of EC50 was calculated using the Phototox v. 2.0 software (ZEBET). Dark viability was measured in parallel under the same conditions without irradiation.

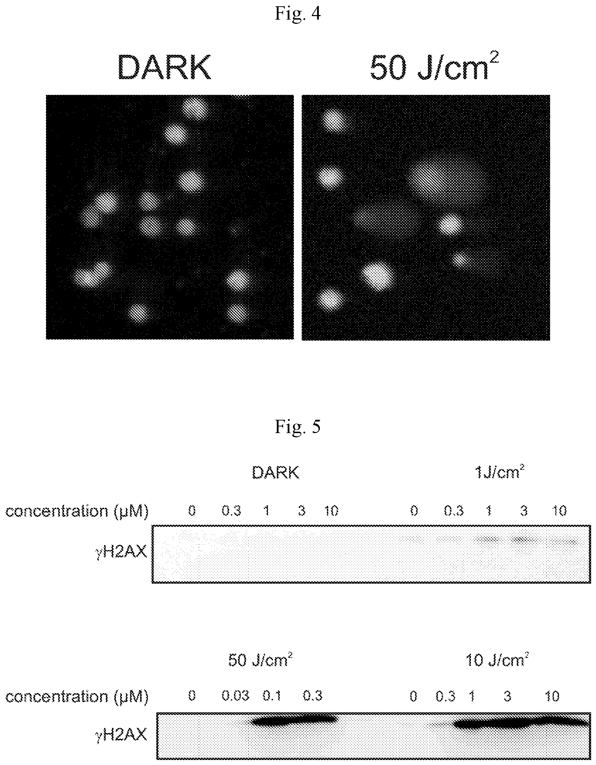

The viability of cells treated with compounds **5, 7** and **10** for 24 h without irradiation was similar to untreated cells, thus concluding that the compounds have no cytotoxicity up to 10 µM concentrations (Figure 4).

### Example 11 Photodynamic treatment

An in-house constructed LED based light source specifically designed for the irradiation of 96-well microplates and Petri dishes (Tomecka et al., CZ302829B6, 2006) was used; a maximal wavelength emission of 414 nm and light intensity set to 20 mW/cm². For photodynamic treatments, cultivation medium was aspirated and replaced by PBS supplemented with 5 mM glucose. Immediately after irradiation, PBS was removed and full DMEM was added to cells. The cultivation lasted for subsequent 24 h. All assays were performed in triplicates and repeated independently three times.

In an initial experiment, G361 cells were treated with the tested compounds for 24 h and then exposed to light (LED source, 414 nm) with a total irradiation dose of 10 J/cm². Viability of cells was quantified 24 h after the irradiation by an MTT assay (Table 3). Subsequently, G361 cells treated with compound 7 were also exposed to total irradiation doses of 1 and 50 J/cm². The obtained results are shown in Table 4.

**Table 3. EC50 of compounds 5, 7 and 10 in irradiated G361 cells (10 J/cm²).**

| **Compound** | **EC50 ± SD (µM)** |
|---|---|
| **5** | 6.213 ± 0.038 |
| **7** | 0.262 ± 0.002 |
| **10** | 2.058 ± 0.082 |

**Table 4. EC50 of compound 7 in G361 cells irradiated with different light energy.**

| | **EC50** ± **SD (µM)** |
|---|---|
| **1 J/cm²** | 3.079 ± 0.155 |
| **10 J/cm²** | 0.262 ± 0.002 |
| **50 J/cm²** | 0.052 ± 0.004 |

### Example 12 Measurement of reactive oxygen species (ROS) production

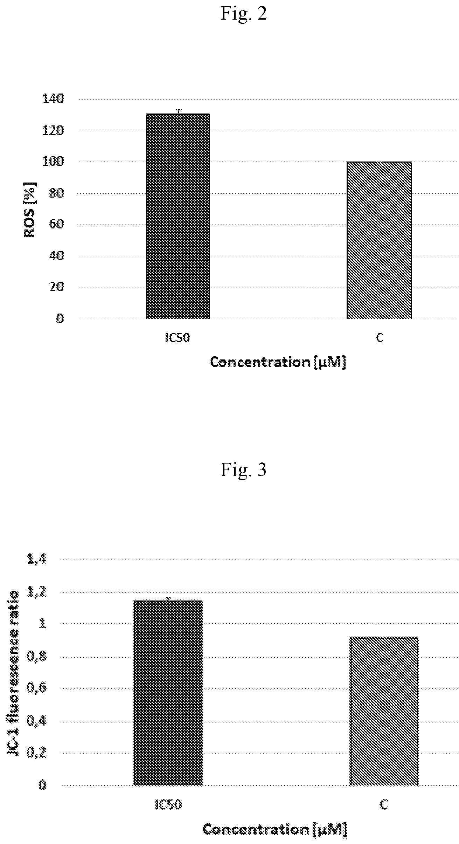

ROS was quantified using a CM-H₂DCFDA (Invitrogen) solution (10 µl 10 µM DCF + 1 ml PBS) and a microplate reader Synergy HT. Right after adding 50 µl of DCF solution, 25 min incubation and irradiation, the ROS production was determined by measuring fluorescence using Synergy HT reader. An excitation wavelength of 485 nm and an emission wavelength of 548 nm were used. ROS production upon treatment with 7 at a concentration corresponding to EC50 (0.052 µM) and irradiation of 50 J/cm2 increased by 30% in comparison to control cells (Figure 2).

### Example 13 Mitochondrial membrane potential change assay

Mitochondrial membrane potential change was monitored by the fluorescent dye JC-1. After 24 h incubation the cells were washed by PBS. Then 50 µl of a JC-1 solution (Sigma-Aldrich) was added (5 mg/ml in DMSO + 5 ml PBS) and cells were incubated at 37 °C and in 5 % CO₂ for 20 min. After incubation the cells were washed by PBS twice. The results were expressed as the ratio of the green fluorescence (excitation wavelength - 485 nm, emission wavelength - 548 nm) and red fluorescence (excitation wavelength - 520 nm, emission wavelength - 590 nm) retained within the cells. JC fluorescence was measured using Synergy HT reader. G361 cells treated with compound **7** and then irradiated showed a decrease in JC-1 polymer aggregation within mitochondria (Figure 4) suggesting its membrane depolarization, which is a characteristic sign of oxidative stress and induction of apoptosis.

### Example 14 Comet assay

Microscope slides were pre-coated with 1% HMP agarose (Qbiogene) dissolved in distilled water. After stiffening, two 1% agarose spots (dissolved in PBS) were made and overlayed with a cover slip. Treated or control G361 cells were trypsinized, rinsed by DMEM and then the mixture of 25 µl of cell suspension and 85 µl of 1% LMP agarose (dissolved in PBS) was added to the microscope slides with agarose gel. The microscope slides were immersed in a lysis buffer with 1% Triton X for 1 h and then placed in an electrophoretic tank and dipped into a cool electrophoresis solution for 40 min. After the electrophoresis (20 V, 350 mA, 20 min), the microscopic slides were immersed in a neutralisation buffer (10 min twice). The samples were then stained by SYBR Green (Invitrogen) for 15 min and scored by the SW Comet Score (TriTek Corp.).

In order to determine the extent of DNA fragmentation caused by a combination of compound **7** and irradiation in G361 cells, comet assay was performed (Figure 4). The cells were divided into four groups according to degree of DNA fragmentation: 0-10%, 11-30%, 31-50% and 50-100%. As expected, compound **7** caused significant DNA fragmentation in comparison with untreated control cells (Table 5).

**Table 5. Degree of DNA fragmentation in G361 cell line treated by compound 7 at 0.052 µM and irradiated with 50 J/cm².**

| **Degree of DNA fragmentation [%]** | **relative number of cells [%]** | |
|---|---|---|
| | **untreated** | **treated** |
| 0-10 | 100.00 ± 0.00 | 62.83 ± 2.72 |
| 11-30 | 0.00 | 30.73 ± 4.07 |
| 31-50 | 0.00 | 4.84 ± 0.65 |
| 51-100 | 0.00 | 1.6 ± 1.59 |

### Example 15 DNA damage monitored by phosphorylation of histone H2A.X

After the treatment, cell lysates were prepared in RIPA buffer, then proteins were separated on SDS-polyacrylamide gels and electroblotted onto nitrocellulose membranes. After blocking, overnight incubation with specific primary antibodies and incubation with peroxidase-conjugated secondary antibodies, the peroxidase activity was detected with SuperSignal West Pico reagents (Thermo Scientific) using a CCD camera LAS-4000 (Fujifilm). All primary antibodies were diluted in PBS containing 5% powdered milk and 0.1% Tween 20. The specific antibodies were purchased from Cell Signaling (anti-PARP, clone 46D11; HRP-linked rabbit anti-mouse immunoglobulin), Sigma Aldrich (anti-α-tubulin, clone DM1A), Millipore (anti-phospho-histone H2A.X, Ser139, clone JBW301).

To independently confirm DNA damage caused by a combination of compound **7** and irradiation in G361 cells, phosphorylation of histone H2A.X at Ser-139 (yH2A.X) was evaluated using immunoblotting analysis (Figure 5). Increased level of yH2A.X is a well-accepted marker of DNA damage. The cells were treated with different doses of compound 7 for 24 h, then either kept in dark or exposed to light with a total irradiation dose of 1, 10 or 50 J/cm² and cultivated for subsequent 24 h. After this period, the cells were harvested and analyzed by immunoblotting. In accordance with abovementioned findings, cells kept either in the dark or without compound **7** did not show any H2A.X phosphorylation, but its level increased both with the compound concentration and with irradiation energy (Figure 5); faint yH2A.X signal was already observed at the dose of 1 J/cm2, while higher irradiation doses (10 and 50 J/cm2) triggered massive increase of yH2A.X.

### Example 16 Antimicrobial activity

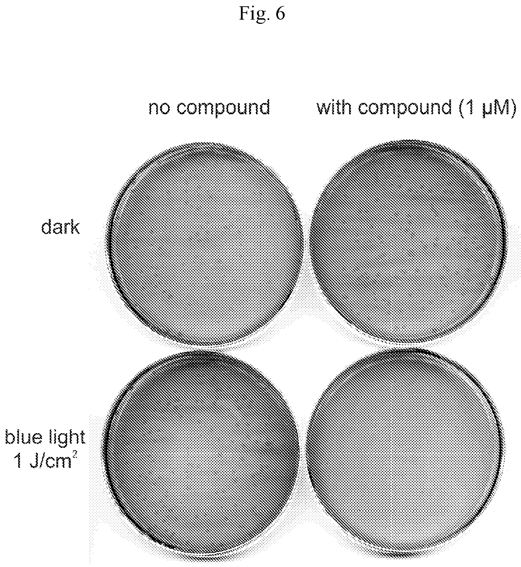

Antimicrobial activity was tested on *Escherichia coli* (DH5alpha).The cells were grown aerobically at 37°C in LB medium. When reached exponential growth phase, the suspension was diluted with fresh LB and supplemented with test compounds or with diluted DMSO (as a control) and seeded on Petri dishes with agar media. After 5 hours the plates were exposed to light from a light emitting diode source, a maximal wavelength emission of 414 nm, light intensity set to 20 mW/cm² (M. Tomecka, et al., CZ302829B6, 2006). The control samples for dark toxicity were not exposed to the light. After irradiation the plates were in the dark incubator at 37 °C for 16 h and then the colonies were counted. The results of the experiments are shown in Figure 6. A strong antibacterial effect by was observed when cells were treated with 1 µM compound and irradiated with 1 J/cm². The antibacterial effect was dose-dependent for both compound concentration and irradiation time.

### Example 17 Dry Capsules

5000 capsules, each of which contains 0.25 g of a compound of the formula **I** as an active ingredient, are prepared as follows:
Composition:

| | |
|---|---|
| Active ingredient | 1250 g |
| Talc | 180 g |
| Wheat starch | 120 g |
| Magnesium stearate | 80 g |
| Lactose | 20 g |

Preparation process: The powdered substances mentioned are pressed through a sieve of mesh width 0.6 mm. Portions of 0.33 g of the mixture are transferred to gelatine capsules with the aid of a capsule-filling machine.

### Example 18 Soft Capsules

5000 soft gelatine capsules, each of which contains 0.05 g of a compound of the formula I as an active ingredient, are prepared as follows:
Composition:

| | |
|---|---|
| Active ingredient | 250 g |
| Lauroglycol | 2 liters |

Preparation process: The powdered active ingredient is suspended in Lauroglykol^{®} (propylene glycol laurate, Gattefossé S.A., Saint Priest, France) and ground in a wet-pulveriser to a particle size of about 1 to 3 µm. Portions of in each case 0.419 g of the mixture are then transferred to soft gelatin capsules by means of a capsule-filling machine.

### Example 19 Soft Capsules

5000 soft gelatin capsules, each of which contains 0.05 g of a compound of the formula **I** as an active ingredient, are prepared as follows:
Composition:

| | |
|---|---|
| Active ingredient | 250 g |
| PEG 400 | 1 liter |
| Tween 80 | 1 liter |

Preparation process: The powdered active ingredient is suspended in PEG 400 (polyethylene glycol of Mr between 380 and about 420, Sigma, Fluka, Aldrich, USA) and Tween^{®} 80 (polyoxyethylene sorbitan monolaurate, Atlas Chem. Inc., Inc., USA, supplied by Sigma, Fluka, Aldrich, USA) and ground in a wet-pulveriser to a particle size of about 1 to 3 µm. Portions of in each case 0.43 g of the mixture are then transferred to soft gelatine capsules by means of a capsule-filling machine.

### Example 20 Gel formulation

The components are given in grams per 100 g.

| Compound | Content |
|---|---|
| Compound 7 | 1.0 g |
| Butylhydroxytoluenum | 0.2 g |
| Butylparaben | 0.2 g |
| Diethyleneglycol monoethyl ether | 10.0 g |
| Silica colloidalis anhydrica | 5.0 g |
| Propylene glycol laurate | 83.6 g |

The gel consistence may be additionally modified by addition of silica colloidalis anhydrica. It is again expected that the transdermal Transcutol P/Lauroglycol FCC system will increase the efficiency of compound 7. Silica colloidalis anhydrica will probably slow down the penetration of the active substance.

### Example 21 Preparation procedure of a skin ointment

The formulation components are given in grams per 200 g:

| Compound | Content |
|---|---|
| Compound 7 | 2.0 g |
| Butylhydroxytoluenum | 0.4 g |
| Butylparaben | 0.4 g |
| Diethyleneglycol monoethyl ether | 20.0 g |
| Glycerol dibehenate | 44.0 g |
| Propylene glycol laurate | 133.2 g |

### Recommended procedure:

Phase A: 2 grams of compound **7** were dissolved in 20 g of Transcutol P while stirring continuously at room temperature in a separate glass or stainless-steel container. The dissolution process may be accelerated by heating the solution to a maximal temperature of 40 °C.

Phase B: 0.4 grams of Nipanox BHT and 0.4 g of Nipabutyl were dissolved while stirring continuously in 133.2 g of Lauroglycol FCC at a temperature of approximately 70 °C in another separate glass or stainless-steel container. The clear oily solution is heated to a temperature of approximately 80 °C and 44 g of Compritol 888 ATO are melted in it while stirring continuously. The clear oily solution is cooled down to approximately 60 °C and during continuous stirring and cooling down is mixed with phase A. The resulting whitish ointment-like substance is divided into approximately 15 gram portions and filled into prearranged plastic containers.

### Example 22 Formulation of a composition for topical application to the skin

A composition for topical application to the skin contains the following ingredients by weight%:

| Active ingredient: | Compound 7 | 0.1% |
|---|---|---|
| Oil phase: | Cetyl alcohol | 5.0% |
| | Glyceryl monostearate | 15.0% |
| | Sorbitan monooleate | 0.3% |
| | Polysorbate 80 USP | 0.3% |
| Aqueous phase: | Methylcellulose 100 cps | 1.0% |
| | Methyl paraben | 0.25% |
| | Propyl paraben | 0.15% |
| | Purified water | q.s. to 100% |

Methyl paraben and propyl paraben were dissolved in hot water and subsequently methylcellulose was dispersed in the hot water. The mixture was chilled at 6 °C until the methylcellulose dissolved. The mixture was then heated to 72 °C and added to the oil phase which was heated to 70 °C while stirring continuously. Compound **7** was added at a temperature of 35 °C and the resulting mixture was stirred continuously until dispersed.

## Claims

1. Conjugated pyrazole-indole derivatives of the general formula I 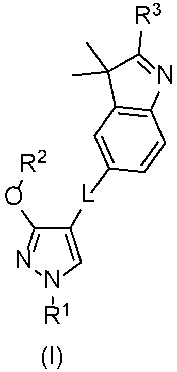 wherein:
L is selected from the group consisting of -CH=CH-, -CH=CH-CH=CH- and -C≡C-;
R¹ is phenyl which can be unsubstituted or optionally substituted with 1 to 2 substituents selected from the group comprising F, methyl and methoxy.
R² is C1-C8 alkyl, preferably C1-C6 alkyl, which can be branched or linear, wherein in the said alkyl one or two carbon atoms may optionally be replaced by one or two heteroatoms selected from O, N and S;
R³ is phenyl, which can be unsubstituted or optionally substituted with 1 to 2 substituents selected from the group consisting of F, Cl, and methoxy.
or pharmaceutically acceptable salts thereof.

2. Conjugated pyrazole-indole derivatives of the general formula I according to claim 1, selected from the group consisting of 5-{[3-(hexyloxy)-1-phenyl-1*H*-pyrazol-4-yl]ethynyl}-3,3-dimethyl-2-phenyl-3*H-*indole, 5-{(*E*)-2-[3-(hexyloxy)-1-phenyl-1*H*-pyrazol-4-yl]ethenyl}-3,3-dimethyl-2-phenyl-3*H*-indole, and 5-{(1*E,*3*E*)-4-[3-(hexyloxy)-1-phenyl-1*H*-pyrazol-4-yl]buta-1,3-dien-1-yl}-3,3-dimethyl-2-phenyl-3*H-*indole.

3. Conjugated pyrazole-indole derivatives of the general formula I according to claim 1 or 2 for use as medicaments.

4. Conjugated pyrazole-indole derivatives of the general formula I according to claim 1 or 2 for use in a method of treatment disorders involving cell proliferation.

5. Conjugated pyrazole-indole derivatives of the general formula I according to claim 1 or 2 for use for inducing apoptosis in mammalian cells *in vitro* or *in vivo.*

6. Conjugated pyrazole-indole derivatives of the general formula I according to claim 1 or 2 for use in photodynamic therapy of cancer.

7. Conjugated pyrazole-indole derivatives of the general formula I according to claim 1 or 2 for use in photodynamic therapy of bacterial infections.

8. A pharmaceutical composition, **characterized in that** it contains at least one conjugated pyrazole-indole derivative of the general formula I according to claim 1 or 2 and a pharmaceutically acceptable carrier.

## Patentansprüche

1. Konjugierte Pyrazol-Indol-Derivate der allgemeinen Formel I 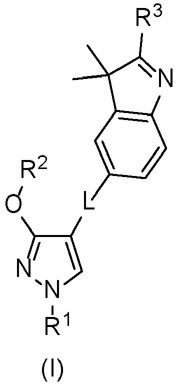 worin:
L ist ausgewählt aus der Gruppe bestehend aus -CH=CH-, -CH=CH-CH=CH- und -C=C-;
R¹ ist Phenyl, das unsubstituiert oder gegebenenfalls mit 1 bis 2 Substituenten, ausgewählt aus der Gruppe bestehend aus F, Methyl und Methoxy, substituiert sein kann,
R² ist C1-C8-Alkyl, vorzugsweise C1-C6-Alkyl, das verzweigt oder linear sein kann, wobei in dem Alkyl ein oder zwei Kohlenstoffatome gegebenenfalls durch ein oder zwei Heteroatome, ausgewählt aus O, N und S, ersetzt sein können;
R³ ist Phenyl, das unsubstituiert oder gegebenenfalls substituiert mit 1 bis 2 Substituenten, ausgewählt aus der Gruppe bestehend aus F, Cl und Methoxy, sein kann.
oder pharmazeutisch verträgliche Salze davon.

2. Konjugierte Pyrazol-Indol-Derivate der allgemeinen Formel I nach Anspruch 1, ausgewählt aus der Gruppe bestehend aus 5-{[3-(Hexyloxy)-1-phenyl-1H-pyrazol-4-yl]ethinyl}-3,3-dimethyl-2-phenyl-3H-indol, 5-{(*E*)-2-[3-(hexyloxy)-1-phenyl-1*H*-pyrazol-4-yl]ethenyl}-3,3-dimethyl-2-phenyl-3*H*-indol und 5-{(1*E*,3*E*)-4-[3-(hexyloxy)-1-phenyl-1*H*-pyrazol-4-yl]buta-1,3-dien-1-yl}-3,3-dimethyl-2-phenyl-3H-indol.

3. Konjugierte Pyrazol-Indol-Derivate der allgemeinen Formel I nach Anspruch 1 oder 2 zur Verwendung als Arzneimittel.

4. Konjugierte Pyrazol-Indol-Derivate der allgemeinen Formel I nach Anspruch 1 oder 2 zur Verwendung in einem Verfahren zur Behandlung von zellproliferation-umfassenden Erkrankungen.

5. Konjugierte Pyrazol-Indol-Derivate der allgemeinen Formel I nach Anspruch 1 oder 2 zur Verwendung zur Induktion von Apoptose in Säugetierzellen *in vitro* oder *in vivo.*

6. Konjugierte Pyrazol-Indol-Derivate der allgemeinen Formel I nach Anspruch 1 oder 2 zur Verwendung in der photodynamischen Therapie von Krebserkrankungen.

7. Konjugierte Pyrazol-Indol-Derivate der allgemeinen Formel I nach Anspruch 1 oder 2 zur Verwendung in der photodynamischen Therapie bakterieller Infektionen.

8. Pharmazeutische Zusammensetzung, **dadurch gekennzeichnet, dass** sie mindestens ein konjugiertes Pyrazol-Indol-Derivat der allgemeinen Formel I nach Anspruch 1 oder 2 und einen pharmazeutisch verträglichen Träger enthält.

## Revendications

1. Dérivés conjugués de pyrazole-indole de formule générale I 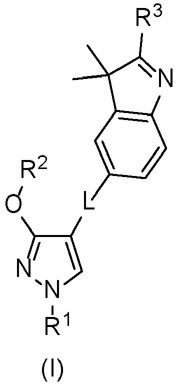 où:
L est choisi dans le groupe consistant en -CH=CH-, -CH=CH-CH=CH- et -C=C- ;
R¹ est phényle qui peut être non substitué ou éventuellement substitué par 1 à 2 substituants choisis dans le groupe comprenant F, méthyle et méthoxy;
R² est alkyle en C1-C8, de préférence alkyle en C1-C6, pouvant être ramifié ou linéaire, où dans ledit alkyle un ou deux atomes de carbonepeuvent éventuellement être remplacés par un ou deux hétéroatomes choisis parmi O, N et S;
R³ est phényle, qui peut être non substitué ou éventuellement substitué par 1 à 2 substituants choisis dans le groupe constitué de F, Cl et méthoxy;
ou leurs sels pharmaceutiquement acceptables.

2. Dérivés conjugués de pyrazole-indole de formule générale I selon la revendication 1, choisis dans le groupe consistant en 5-{[3-(hexyloxy)-1-phényl-1*H*-pyrazol-4-yl]éthynyl}-3,3-diméthyl-2-phényl-3*H-*indole, 5-{(*E*)-2-[3-(hexyloxy)-1-phényl-1*H*-pyrazol-4-yl]éthényl}-3,3-diméthyl-2-phényl-3*H*-indole et 5-{(1*E*,3*E*)-4-[3-(hexyloxy)-1-phényl-1*H*-pyrazol-4-yl]buta-1,3-diène-1-yle}-3,3-diméthyl-2-phényl-3H-indole.

3. Dérivés conjugués de pyrazole-indole de formule générale I selon la revendication 1 ou 2 pour utilisation comme médicaments.

4. Dérivés conjugués de pyrazole-indole de formule générale I selon la revendication 1 ou 2 pour utilisation dans une méthode de traitement de troubles impliquant la prolifération cellulaire.

5. Dérivés conjugués de pyrazole-indole de formule générale I selon la revendication 1 ou 2 pour utilisation pour induire l'apoptose dans des cellules de mammifères *in vitro* ou *in vivo.*

6. Dérivés conjugués de pyrazole-indole de formule générale I selon la revendication 1 ou 2 pour utilisation en thérapie photodynamique du cancer.

7. Dérivés conjugués de pyrazole-indole de formule générale I selon la revendication 1 ou 2 pour utilisation en thérapie photodynamique d'infections bactériennes.

8. Composition pharmaceutique, **caractérisée en ce qu'**elle contient au moins un dérivé conjugué de pyrazole-indole de formule générale I selon la revendication 1 ou 2 et un support pharmaceutiquement acceptable.
